(19) Europäisches Patentamt

European Patent Office

Office européen des brevets

(11) **EP 1 614 388 A1**

(12) **EUROPEAN PATENT APPLICATION**

(43) Date of publication:
**11.01.2006 Bulletin 2006/02**

(51) Int Cl.:
*A61B 8/08* (2006.01)

(21) Application number: **04291758.3**

(22) Date of filing: **09.07.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(71) Applicant: **INSTITUT NATIONAL DE LA SANTE ET DE LA RECHERCHE MEDICALE**
**75654 Paris Cedex 13 (FR)**

(72) Inventors:
• **Chapelon, Jean-Yves**
**69100 Villeurbanne (FR)**
• **Souchon, Rémi**
**38200 vienne (FR)**

(74) Representative: **Hirsch & Associés**
**58, avenue Marceau**
**75008 Paris (FR)**

(54) **Method for performing elastography**

(57)  A method for performing elastography is provided, comprising the following steps. First, emitting a first signal towards tissue and recording an echoed signal in response. Next, applying a strain on the tissue, such as a compression or an expansion. Then, emitting a second signal towards the tissue under strain, said second signal being a compressed (or stretched) version of the first emitted signal in the time domain, and recording an echoed signal in response. Then processing the second echoed signal by stretching (or compressing) it in the time domain by a factor matched to the compression (or stretching) factor that was applied to the second emitted signal. Finally, cross-correlating the first echoed signal and the processed second echoed signal to provide an elastographic image of said tissue.

The method of the invention uses different emitted signals before and during strain applied to the tissue. The image quality in elastography can therefore be considerably improved.

*Fig 1*

EP 1 614 388 A1

**Description**

The invention relates generally to a method for performing elastographic diagnosis of a target body.

Ultrasonic elastography is a system for measuring and imaging elastic modulus and compressibility distributions in an elastic tissue. This system is typically based on external compression of a target body, and utilizes one or more transducers, acting as or with a compressor, to generate pre- and post- compression sonic pulses and receive the resulting echo sequences from within the target body. The pre- and post-compression echo sequence pairs may then be cross-correlated or matched to determine the strain along the path of the sonic pulses, and preferably to yield a strain profile of the target body. This strain profile may then be converted into a compressibility profile or elastogram by measuring the stress imposed by the compressing device and calculating the elastic moduli based on the stress and the strain profile.

Elastography has become a medical imaging technique involving evaluating internal deformation undergone by tissue during compression in order to determine elasticity of said tissue and therefore eventually detect abnormal masses. This technique can be used for cancer screening for instance.

Elastography method and apparatus are described in patent US-A-5 474 070.

What we refer to by the term elastographic image is the strain distribution on a spatial basis. The spatial basis may be defined as lateral scanning versus depth. The depth can be given by time travel of the signals, i.e. time range between the pulse emission and the echoed reception. Lateral scanning represents the surface portion of tissue to be analyzed. The image therefore gives a two-dimensional representation of a given surface of tissue.

Image quality in elastography can be estimated by quantities such as elastographic signal-to-noise ratio (SNRe) and axial resolution (Ra). SNRe is defined as the mean to standard deviation ratio of the measured strain in an area of uniform strain. Ra is defined as the smallest distance between two resolvable targets.

It has been shown that high cross-correlation values are required between the pre-compression signal and the post-compression signal in order to obtain a precise estimation of the displacement, hence precise strain estimates.

In elastography, correlation is degraded by two factors: increasing strain and decreasing sonographic signal-to-noise ratio (SNRs). Because correlation degrades with increasing strain, there exists an upper bound that prevents elastographic acquisition for strains greater than about 1-2%. Stretching of the post-compression backscattered signal before time delay estimation has been proposed but the method only partially overcomes this degradation. Regarding the post-compression signal stretching, it can be referred to the publications "Noise reduction in elastograms using temporal stretching with multicompression averaging", by T. Varghese, J. Ophir and I. Cespedes, Ultrasound in Med & Biol, Vol. 22, No. 8, pp 1043-1052, 1996; "An adaptive strain estimator for elastography", by SK. Alam, J. Ophir, E. Konofagou, IEEE Trans Ultrason Ferroelectr Freq Control, Vol. 45, No. 2, pp 461-472, 1998 and to patents US-A-6 514 204 and US-A-6 277 074.

SNRs is defined as the ratio of the ultrasonic signal power over the noise power at a given spatial location. High SNRs is required to achieve high correlation. It can be obtained by increasing the amplitude or the duration of the emitted signal. In biological tissues the maximal signal amplitude is limited by the Mechanical Index (MI) that can be applied before cavitation-induced damage starts to occur. Most ultrasound scanners already operate at this level, so that pulse amplitude cannot be further increased. Increasing pulse duration using bursts provides increased SNRs but degrades the axial resolution of the ultrasonic system.

This problem of image quality was previously identified in sonographic imaging. Usual sonography systems have chosen to use short pulse waves to favor axial resolution, and perform signal processing to deal with SNRs.

Alternatively, emitting long coded excitation signal was shown to increase SNRs in conventional sonography and in Doppler imaging without impairing spatial resolution. Regarding long coded ultrasonic excitation signals, it can be referred to the publication "Coded excitation for diagnostic ultrasound: A system developer's perspective", by RY. Chiao and X. Hao, Proceeding IEEE Ultrasonics Symposium 2003, Vol. 1, pp 437-448 and to patent US-A-6 213 947.

It is an object of the invention to propose an elastography method that totally overcomes the image degradation due to strain, and that minimizes image degradation due to SNRs without impairing axial resolution.

According to the invention a new elastography method is provided using different emitted signals before and during compression of the tissues.

In particular, the invention concerns a method for performing elastography, the method comprising the steps of:

a) emitting a first signal $p_1(t)$ towards tissue, where t denotes the time;
b) recording a first echoed signal $s_1(t)$ in response to said first emitted signal;
c) applying a strain on the tissue;
d) emitting a second signal $p_2(t)$ towards the tissue under strain, said second emitted signal being linked to said first emitted signal with the following relation:

$$p_2(t) = p_1(\alpha t),$$

with $\alpha = 1/(1+\varepsilon)$, $\varepsilon$ being the uniform strain applied to the tissue;
e) recording a second echoed signal $s_2(t)$ in response to said second emitted signal ;
f) processing said second echoed signal according to the following relation: $S_2''(t) = s_2(t/\alpha)$;
g) cross-correlating the first echoed signal $s_1(t)$ and the processed second echoed signal $s''_2(t)$ to provide an elastographic image of said tissue.

According to embodiments of the invention, the method comprises one or several of the following features:

- the strain applied to the tissue in step c) is negative, providing a compression of said tissue;
- the strain applied to the tissue in step c) is positive, providing an expansion of said tissue;
- the first and second emitted signals are pulse signals;
- the first and second emitted signals are burst signals;

According to one embodiment, the method further comprises the step of coding the emitted signals.
According to one embodiment, the second signal $p_2(t)$ is emitted with a predetermined value of $\alpha$ in step d), providing an elastographic image in step g), the method further comprising the steps of:

h) calculating a local strain $\varepsilon$ applied on tissue from the results of said elastographic image;
i) adjusting the value of $\alpha$ according to the calculated local strain $\varepsilon$;
j) repeating steps d) to g) with an adjusted value of $\alpha$.

According to one embodiment, steps h) to j) are repeated iteratively until the adjusted value of $\alpha$ is substantially equal to the value applied to the second emitted signal $p_2(t)$.
The invention also concerns a computer system for performing the elastography method according to the invention, comprising:

- computer-implemented software to link the second signal to be emitted to the first emitted signal;
- computer-implemented software to cross-correlate the echoed signals. According to embodiments, the computer system may further comprises:
- computer-implemented software to calculate a local strain $\varepsilon$ applied on tissue and adjust the value of $\alpha$ accordingly; and/or
- computer-implemented software to code the emitted signals.

Other characteristics and advantages of the invention will appear on reading the following detailed description of some embodiments of the invention given solely as an example and taken in connection with the accompanying drawings in which:

- figure 1 is a graph showing pre- and post-compression short pulse emitted signals;
- figure 2 is a graph showing pre- and post-compression Barker coded emitted signals.

The invention concerns a method where the pre- and post-compression emitted signals have different characteristics, these characteristics being chosen depending on the strain to be measured.
According to the invention, a new elastography method is provided comprising the following steps. First, emitting a first signal towards tissue and recording a first echoed signal in response to said first signal. Next, applying a strain on the tissue, such as a compression or an expansion, preferably of a known value. The strain applied to the tissue could also alternate compression and expansion. Then, emitting a second signal towards the tissue under strain, said second signal being a compressed (or stretched) version of the first emitted signal in the time domain, and recording a second echoed signal in response to said second signal. Then processing the second echoed signal by stretching (or compressing) it in the time domain by a factor matched to the compression or stretching factor that was applied to the second emitted signal. Finally, cross-correlating the first echoed signal and the processed second echoed signal to provide an elastographic image of said tissue.
The time-domain stretching or compression of the second emitted signal depends on the strain applied to the tissue, i.e. if the tissue is compressed (respectively stretched by expansion), the second emitted signal will be a compressed version (respectively a stretched version) of the first emitted signal.

According to the invention, the elastography method uses different emitted signals before and during strain applied to the tissue. In particular, the invention concerns a method for which the shape of the post-compression signal is a compressed (or stretched) version of the pre-strain signal. The compression (or stretching) factor $\alpha$ can be matched to an estimate of the applied strain $\varepsilon$. The pre-and post-compression signals can be short pulses, bursts or coded signals.

According to the invention, an ultrasonic signal is emitted towards tissue, being firstly at rest. As well known from sonography, an echoed signal is returned from the tissue. Such echoed signal can be recorded and analyzed to provide information on the tissue condition.

The ultrasonic pulse p(t) emitted towards tissue can be modeled as follows:

$$p(t) = e(t) * h(t)$$

where t denotes time, and
with, e(t) being the electric pulse applied to the ultrasonic transducer
h(t) being the transducer impulse response
the symbol * being the convolution product

If noise is neglected, the echoed signal s(t) can be modeled as follows:

$$s(t) = p(t) * m(t)$$

with, m(t) being the reflectivity function of the scatterer distribution that model the tissue.

The echoed signal recorded before strain is applied to the tissue is

$$s_1(t) = p_1(t) * m(t);$$

and the echoed signal recorded while applying a strain to the tissue is

$$s_2(t) = p_2(t) * m(\alpha t);$$

with $\alpha = 1/(1+\varepsilon)$ linked to the uniform strain $\varepsilon$ applied to the tissue. If $\varepsilon$ is positive, the tissue is expanded and if $\varepsilon$ is negative, the tissue is compressed. $p_1(t)$ and $p_2(t)$ are respectively the pre- and post-compression emitted ultrasonic pulses.

The cross-correlation in the frequency domain of said echoed signals before and during strain is formulated as such:

$$C(f) = S_1(f)\, S_2^{*}(f)$$

with $S_1(f)$ and $S_2(f)$ being the Fourier Transform of the respective echoed signals $s_1(t)$ and $s_2(t)$, and * being the complex conjugation.

In conventional elastography, the emitted pre and post-compression pulses $p_1(t)$ and $p_2(t)$ are identical. The cross-correlation function can be expressed as follows:

$$C(f) = (1/|\alpha|)\, \|P_1(f)\|^2\, M(f)\, M^{*}(f/\alpha)$$

with M(f) being the Fourier Transform of the reflectivity function m(t). Correlation degrades with increasing strain because the M(f)M*(f/$\alpha$) product is lower that the autocorrelation function $\|M(f)\|^2$ of the tissue.

Uniform or local stretching was proposed to recover the autocorrelation function of the tissue. It can be referred to the previously cited publications of T. Varghese, 1996; SK. Alam, 1998 and patents US-A-6 514 204 and US-A-6 277 074.

The principle of these methods is to stretch the post-compression echoed signal $s_2(t)$ by a factor $\alpha$ in the time

domain, i.e. to define $s_2'(t)$ as follows:

$$s_2'(t) = s_2(t/\alpha) = p_2(t/\alpha)*m(t)$$

Then the cross-correlation between the pre-compression signal s1(t) and the stretched post-compression signal $s_2'(t)$ is, in the frequency domain:

$$C(f) = (1/|\alpha|) P_1(f) P_1^*(\alpha f) ||M(f)||^2$$

This method recovers the autocorrelation function of the tissues but at the cost of degrading the autocorrelation function of the transmitted pulse $||P_1(f)||^2$. This is because the point-spread function (PSF) is also stretched with this method.

However, according to the invention, the pre- and post- strain signals are not identical, i.e. the duration of the second emitted pulse is stretched or compressed compared to the duration of the first emitted pulse.

The post-compression pulse $p_2(t)$ is compressed in the time domain compared to the pre-compression pulse $p_1(t)$ and can be expressed as follows:

$$p_2(t) = p_1(\alpha t).$$

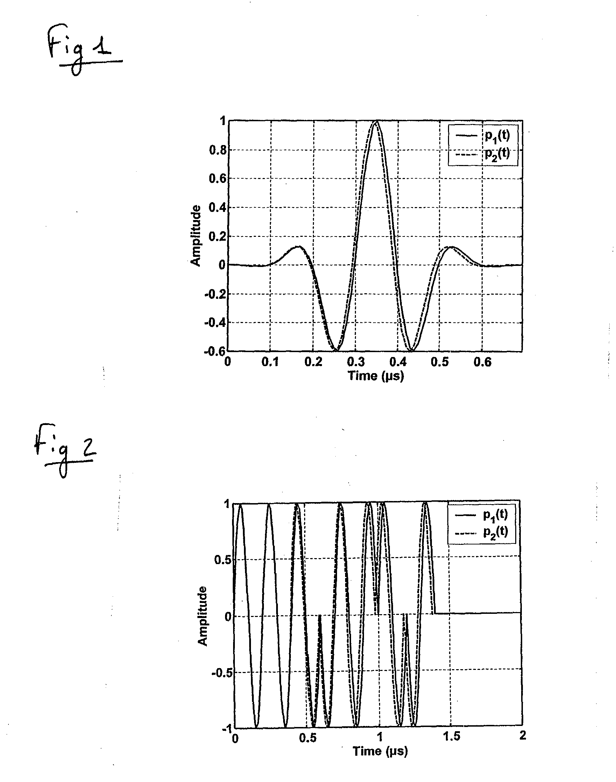

Figure 1 shows an example of emitted signals $p_1(t)$ and $p_2(t)$ with a factor $\alpha=1.02$, corresponding to an applied compression strain $\varepsilon$ of 2% on the tissue of the target body. The method according to the invention makes it possible to improve greatly the signal to noise ratio through an improvement in the correlation between the pre- and post-compression echoed signals.

In the example of figure 1, an ultrasonic transducer was used to provide pulsed excitations with a central frequency of 5 MHz. It can be seen that the signal $p_2(t)$ is a compressed version in the time domain of $p_1(t)$.

The method creates a time-domain compression (or expansion) of the PSF (Point Spread Function) of the imaging system, which is then restored by numerical stretching (or compression) of the echoed signal, as shown below.

The post-compression signal becomes: $s_2(t) = p_1(\alpha t) * m(\alpha t)$;

Then global or adaptive stretching of the post-compression signal is used to obtain a stretched version $s_2''$ of the post-compression echoed signal:

$$s_2''(t) = s_2(t/\alpha) = p_1(t)*m(t) = s_1(t)$$

Then the cross-correlation between the pre-compression signal s1(t) and the stretched post-compression signal $s_2''(t)$ can be expressed as:

$$C(f) = S_1(f)S_2''^*(f) = ||S_1(f)||^2 = ||P_1(f)||^2 ||M(f)||^2$$

It can be noted that the cross-correlation function is equal to auto-correlation function of the pre-compression pulse. Therefore, the decorrelation noise due to the strain applied to the tissue is totally eliminated. The image quality in elastography can therefore be considerably improved by such method according to the invention.

The calculation was made regardless of the shape of the emitted signals, which can either be short pulses, bursts, or coded signals.

The strain applied to the tissue may be a compression or an expansion, without impacting on the method according to the invention. The terms pre-compression, post-compression, global stretching and others, can be changed into pre-stretching, post-stretching and global compressing without changing the previous equations, as the value of the factor $\alpha$ depends on the type of strain applied to the tissue.

According to an embodiment of the invention, the image quality can be improved by adjusting the value $\alpha$ to a value as close as possible to the real strain $\varepsilon$ applied. First, the method according to the invention is conducted by emitting a second signal $p_2(t)$ with a predetermined value of $\alpha$, as illustrated on figure 1. A first elastographic image is provided and the local strain $\varepsilon$ applied on tissue can be calculated from said first elastographic image. The value of $\alpha$

can therefore be adjusted to the calculated strain $\varepsilon$ and further second signals $p_2(t)$ can be emitted with an adjusted values of $\alpha$. The emitting of a post-strain signal $p_2(t)$ can be repeated iteratively until the calculated adjusted value of $\alpha$ is substantially equal to the value applied to the second emitted signal $p_2(t)$.

According to an embodiment of the invention, the eventual use of coded excitation allows further improvement in image quality in areas where the sonographic signal-to-noise ratio (SNRs) is low, without any loss in axial resolution.

In this case, the pre-compression emitted signal $p_1(t)$ can be any of the coded signals that can be used for sonography, such as a frequency-modulated chirp, a Barker code, a Golay complementary series or any pseudo-random sequences whose auto-correlation function is a Dirac function. The post-compression emitted signal $p_2(t)$ is derived from the coded pre-compression emitted signal $p_1(t)$ using equation $p_2(t)=p_1(\alpha t)$.

Figure 2 shows an example of pre-compression emitted coded signal $p_1(t)$ and matched post-compression emitted signal $p_2(t)$ designed for an estimated compression strain of 2% ($\alpha$=1.02) and an ultrasonic transducer with a central frequency of 5 MHz, using a Barker code of length 7. The signal $p_2(t)$ is a compressed version in the time domain of $p_1(t)$.

Combining coded excitation with the invention has great potential because the two major causes of noise in displacement estimates in elastography, namely strain-induced decorrelation and SNRs-induced decorrelation, are significantly decreased.

Moreover, using coded excitation in elastography can be very simple as no matched filtering is mandatory on echoed signals before the cross-correlation of the echoed signals is estimated. Cross-correlation of the first echoed signal $s_1(t)$ and the processed second echoed signal $s''_2(t)$ can be conducted on coded signal without impacting the results of the elastographic image

The invention also relates to a computer system able to implement the method according to the invention. The computer system thereof comprises computer-implemented software to link the second signal to be emitted to the first emitted signal, and computer-implemented software to cross-correlate the echoed signals.

The computer system of the invention is adapted to handle the signal processing in order to define the parameters of the post-compression signal $p_2(t)$ to be emitted responding to the criteria of the first or second embodiment of the method of the invention. In particular, the computer comprises hardware or computer-implemented software to stretch or compress the second signal pulse compared to the first signal pulse.

The computer system may also include computer-implemented software to calculate a local strain $\varepsilon$ applied on tissue and adjust the value of $\alpha$ accordingly.

The computer system may also include computer-implemented software to code the emitted signals. The computer system may include software code generator or controlled hardware code generator.

Conventional equipment may be use to implement the elastography method according to the invention. The equipment may comprise a computer, a transmitter emitting signals according to the invention towards tissue and a receiver of echoed signals from the tissue, means for applying a strain on tissue, such as a hand-held compressor or a compressor activated by a motor controlled by the computer. The computer may be linked to a cross-correlator receiving digitalized signal of the emitted and echoed signals towards and from the tissue. A monitor may display the elastographic image. Such an equipment is described in patent US-A-5 474 070.

**Claims**

1. A method for performing elastography, the method comprising the steps of:

   a) emitting a first signal $p_1(t)$ towards tissue, where t denotes the time;
   b) recording a first echoed signal $s_1(t)$ in response to said first emitted signal;
   c) applying a strain on the tissue;
   d) emitting a second signal $p_2(t)$ towards the tissue under strain, said second emitted signal being linked to said first emitted signal with the following relation:

$$p_2(t) = p_1(\alpha t),$$

   with $\alpha= 1/(1+\varepsilon)$, $\varepsilon$ being the uniform strain applied to the tissue;
   e) recording a second echoed signal $s_2(t)$ in response to said second emitted signal ;
   f) processing said second echoed signal according to the following relation:

$$s_2''(t) = s_2(t/\alpha);$$

    g) cross-correlating the first echoed signal $s_1(t)$ and the processed second echoed signal $s''_2(t)$ to provide an elastographic image of said tissue.

**2.** The method according to claim 1, wherein the strain applied to the tissue in step c) is negative, providing a compression of said tissue.

**3.** The method according to claim 1, wherein the strain applied to the tissue in step c) is positive, providing an expansion of said tissue.

**4.** The method according to any one of claims 1 to 3, wherein the first and second emitted signals are pulse signals.

**5.** The method according to any one of claims 1 to 3, wherein the first and second emitted signals are burst signals.

**6.** The method according to any one of claims 1 to 5, further comprising the step of coding the emitted signals.

**7.** The method according to any one of claims 1 to 6, wherein the second signal $p_2(t)$ is emitted with a predetermined value of $\alpha$ in step d), providing an elastographic image in step g) ;
the method further comprising the steps of:

    h) calculating a local strain $\varepsilon$ applied on tissue from the results of said elastographic image;
    i) adjusting the value of $\alpha$ according to the calculated local strain $\varepsilon$;
    j) repeating steps d) to g) with an adjusted value of $\alpha$.

**8.** The method according to claim 7, wherein steps h) to j) are repeated iteratively until the adjusted value of $\alpha$ is substantially equal to the value applied to the second emitted signal $p_2(t)$.

**9.** A computer system for performing the elastography method according to any one of claims 1 to 8, comprising:

    - computer-implemented software to link the second signal to be emitted to the first emitted signal;
    - computer-implemented software to cross-correlate the echoed signals.

**10.** The computer system according to claim 9, further comprising:

    - computer-implemented software to calculate a local strain $\varepsilon$ applied on tissue and adjust the value of $\alpha$ accordingly.

**11.** The computer system according to claim 9 or 10, further comprising:

    - computer-implemented software to code the emitted signals.

Fig 1

Fig 2

**European Patent Office**

EUROPEAN SEARCH REPORT

Application Number

EP 04 29 1758

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,A | ALAM S K ET AL: "AN ADAPTIVE STRAIN ESTIMATOR FOR ELASTOGRAPHY" IEEE TRANSACTIONS ON ULTRASONICS, FERROELECTRICS AND FREQUENCY CONTROL, IEEE INC. NEW.YORK, US, vol. 45, no. 2, 1 March 1998 (1998-03-01), pages 461-472, XP000775484 ISSN: 0885-3010 * page 461 - page 464, paragraph II * * figures 1,2,5,6 * | 1-11 | A61B8/08 |
| A | BRUSSEAU E ET AL: "Axial strain imaging of intravascular data: results on polyvinyl alcohol cryogel phantoms and carotid artery" ULTRASOUND IN MEDICINE AND BIOLOGY, NEW YORK, NY, US, vol. 27, no. 12, December 2001 (2001-12), pages 1631-1642, XP004336367 ISSN: 0301-5629 * page 1634, column 1, paragraph 2 - page 1635, column 1, paragraph 3 * | 1-11 | |
| A | M. BILGEN: "Dynamics of errors in 3D motion estimation and implications for strain-tensor imaging in acoustic elastography" PHYSICS IN MEDICINE AND BIOLOGY, vol. 45, June 2000 (2000-06), pages 1565-1578, XP002305007 * page 1567, line 29 - line 34 * | 1 | |

TECHNICAL FIELDS SEARCHED (Int.Cl.7)

A61B
G01N
G01S

-/--

The present search report has been drawn up for all claims

| Place of search | Date of completion of the search | Examiner |
|---|---|---|
| The Hague | 11 November 2004 | Dydenko, I |

**European Patent
Office**

**EUROPEAN SEARCH REPORT**

Application Number

EP 04 29 1758

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | R. SOUCHON, J.-C. BERA, A. POUSSE, J.-Y. CHAPELON: "Improvement in elastographic signal-to-noise ratio and resolution using coded excitation in elastography" 147TH MEETING OF THE ACOUSTICAL SOCIETY OF AMERICA, 24 May 2004 (2004-05-24), page 2412, XP002305008 * the whole document * | 5,6,11 | |
| D,A | US 6 213 947 B1 (PHILLIPS PATRICK) 10 April 2001 (2001-04-10) * column 3, line 32 - column 11, line 3; figures 1-4 * | 5,6,11 | |
| A | PEDERSEN M H ET AL: "Clinical evaluation of chirp-coded excitation in medical ultrasound" ULTRASOUND IN MEDICINE AND BIOLOGY, NEW YORK, NY, US, vol. 29, no. 6, June 2003 (2003-06), pages 895-905, XP004434875 ISSN: 0301-5629 * page 896, column 1, paragraph 4 - page 899, column 1, paragraph 2 * * figures 1-4 * | 5,11 | |
| A | JUNRU WU ET AL: "TEMPERATURE ELEVATION IN TISSUES GENERATED BY FINITE-AMPLITUDE TONE BURSTS OF ULTRASOUND" JOURNAL OF THE ACOUSTICAL SOCIETY OF AMERICA, AMERICAN INSTITUTE OF PHYSICS. NEW YORK, US, vol. 88, no. 3, 1 September 1990 (1990-09-01), pages 1562-1577, XP000371617 ISSN: 0001-4966 * page 1564 - page 1566, paragraph A-B * | 6 | TECHNICAL FIELDS SEARCHED (Int.Cl.7) |

The present search report has been drawn up for all claims

| Place of search | Date of completion of the search | Examiner |
|---|---|---|
| The Hague | 11 November 2004 | Dydenko, I |

CATEGORY OF CITED DOCUMENTS

X : particularly relevant if taken alone
Y : particularly relevant if combined with another
　　document of the same category
A : technological background
O : non-written disclosure
P : intermediate document

T : theory or principle underlying the invention
E : earlier patent document, but published on, or
　　after the filing date
D : document cited in the application
L : document cited for other reasons

&　: member of the same patent family, corresponding
　　document

EPO FORM 1503 03.82 (P04C01)

**ANNEX TO THE EUROPEAN SEARCH REPORT
ON EUROPEAN PATENT APPLICATION NO.**

EP 04 29 1758

This annex lists the patent family members relating to the patent documents cited in the above-mentioned European search report.
The members are as contained in the European Patent Office EDP file on
The European Patent Office is in no way liable for these particulars which are merely given for the purpose of information.

11-11-2004

| Patent document cited in search report | | Publication date | Patent family member(s) | | Publication date |
|---|---|---|---|---|---|
| US 6213947 | B1 | 10-04-2001 | AU | 4037500 A | 16-10-2000 |
| | | | DE | 10084420 T0 | 06-06-2002 |
| | | | JP | 2002539877 T | 26-11-2002 |
| | | | WO | 0057769 A2 | 05-10-2000 |

EPO FORM P0459